# EUROPEAN PATENT APPLICATION

(11) **EP 4 257 687 A1**
(43) Date of publication of application: **11.10.2023**
(21) Application number: 21898178.5
(22) Date of filing: 30.11.2021
(51) Int. Cl.: C12N 15/61, C12N 1/15, C12N 1/19, C12N 1/20, C12N 1/21, C12N 5/10, C12N 9/90, C12N 11/00, C12N 11/08, C12N 15/31, C12N 15/63, C12P 19/02

(54) **NOVEL L-RHAMNOSE ISOMERASE**

(30) Priority: 30.11.2020 JP 2020198419
(71) Applicant: National University Corporation Kagawa University, Takamatsu-shi, Kagawa 760-8521 (JP)
(72) Inventor: AKIMITSU, Kazuya, Takamatsu-shi, Kagawa 760-8521 (JP); IZUMORI, Ken, Takamatsu-shi, Kagawa 760-8521 (JP); YOSHIHARA, Akihide, Takamatsu-shi, Kagawa 760-8521 (JP); KATO, Shiro, Takamatsu-shi, Kagawa 760-8521 (JP); MOCHIZUKI, Susumu, Takamatsu-shi, Kagawa 760-8521 (JP); YOSHIDA, Hiromi, Takamatsu-shi, Kagawa 760-8521 (JP); KAMITORI, Shigehiro, Takamatsu-shi, Kagawa 760-8521 (JP)
(74) Representative: TBK
(86) International application number: PCT/JP2021/043758
(87) International publication number: WO 2022/114207

(57) **Abstract**

Problem: to provide a highly safe and highly heat-resistant L-rhamnose isomerase usable in the food industry, a microorganism for producing the same, and a method of producing an aldose or ketose.

Solution: an L-rhamnose isomerase derived from a microorganism belonging to the genus Enterobacter, having a subunit molecular mass of 47 kDa as measured by SDS-PAGE, and having the following substrate specificities (A) and (B).
(A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
(B) having reactivity with L-rhamnose, L-lyxose, L-mannose, D-allose, D-ribose, and L-talose.

## Description

### TECHNICAL FIELD

The present invention relates to a novel L-rhamnose isomerase, a method of producing the same, a microorganism for producing the same, a DNA encoding the enzyme, a recombinant vector and a transformed host cell containing the DNA, an immobilized enzyme using the enzyme, and a method of producing a ketose or an aldose with the L-rhamnose isomerase.

### BACKGROUND ART

The term "rare sugar" means, according to the definition by-International Society of Rare Sugars, "rarely occurring sugar in the natural world", in other words, a monosaccharide that exists only in a small amount in the natural world.

As a monosaccharide (tetrose) having 4 carbon atoms, there are 4 aldoses, two ketoses, and three sugar alcohols. As a monosaccharide (pentose) having 5 carbon atoms, there are 8 aldoses, 4 ketoses, and 4 sugar alcohols. As a monosaccharide (hexose) having 6 carbon atoms, there are 34 kinds in total including 16 aldoses, 8 ketoses, and 10 sugar alcohols. As a monosaccharide (heptose) having 7 carbon atoms, there are 32 aldoheptoses, 16 ketoheptoses, and 16 heptitols, each having 7 carbon atoms.

Among these, 6 aldoses exist in a large amount in the natural world and they are D-glucose, D-galactose, D-mannose, D-ribose, D-xylose, and L-arabinose. The other aldoses such as D-allose are defined as a rare sugar. Examples of aldoses defined as a rare sugar include L-allose, L-gulose, L-glucose, L-galactose, L-altrose, L-idose, L-mannose, L-talose, D-talose, D-idose, D-altrose, D-gulose, and D-arose. With regard to the ketoses, D-fructose exists in a large amount in the natural world but the other ketoses do not exist in a large amount in the natural world so that they may be defined as a rare sugar. Examples of the ketoses defined as a rare sugar include D-allulose, D-tagatose, D-sorbose, L-fructose, L-allulose, L-tagatose, and L-sorbose.

Recently, there has been established a mass production technology of D-allulose (another name: D-psicose) which will be a basic raw material for the production of all rare sugars and this makes it possible to produce rare sugars difficult to obtain. Furthermore, due to an enzyme reaction, D-allulose is presumed to play a central role in the production of novel rare sugars such as D-allose.

D-allose is an aldose which is an isomer of D-glucose and is different from only in the OH group direction of the third carbon atom. D-allose is a rare sugar monosaccharide known also as an isomer of D-allulose which is a ketose. The followings are known to have D-allose as an active ingredient: a pharmaceutical composition (Patent Document 1) for the treatment of kidney diseases selected from acute renal failure and uremia, a drug (Patent Document 2) for delaying the onset or progression of dyskinesia caused by amyotrophic lateral sclerosis, a blood pressure elevation inhibitor (Patent Document 3), an agent (Patent Document 4) characterized by being used for the suppression of angiogenesis, a T lymphocyte proliferation inhibitor (Patent Document 5), and a peritoneal deterioration inhibitor (Patent Document 6) to be used after added to a peritoneal dialytic fluid. There have recently been published inventions relating to an antitumor effect (Patent Document 7) of it being taken in renal cell carcinoma cells and a strong antitumor effect (Patent Document 8) against human urothelial carcinoma cells. Due to such characteristics, D-allose has attracted attentions in the pharmaceutical field as a next-generation core material and there is a demand for the establishment of a mass production technology of D-allose following that of D-allulose.

As a method of producing D-allose by using an enzyme produced by a microorganism, the present inventors developed a technology (Non-Patent Document 1) of producing allose from allulose by using an L-rhamnose isomerase isolated from Pseudomonas stutzeri. An L-rhamnose isomerase is an enzyme that catalyzes the reversal isomerization reaction between L-rhamnose and L-rhamnurose. It has been revealed that the L-rhamnose isomerase derived from P. stutzeri has wide substrate specificities and can act on not only between L-rhamnose and L-rhamnurose but also between L-lyxose and L-xylulose, L-mannose and L-fructose, D-gulose and D-sorbose, D-ribose and D-ribulose, D-allose and D-allulose, and L-talose and L-tagatose. Such wide substrate specificities have made it possible to produce various rare aldoses and ketoses on Izumoring, especially the production of D-allose from D-allulose by conversion.

### Prior Art Documents

### Patent Documents

Patent Document 1: Japanese Patent No. 5330976
Patent Document 2: Japanese Patent No. 5317055
Patent Document 3: Japanese Patent No. 5158779
Patent Document 4: Japanese Patent No. 4943839
Patent Document 5: Japanese Patent No. 4724824
Patent Document 6: Japanese Patent Laid-Open No. 2009-269887
Patent Document 7: WO2020/189000
Patent Document 8: WO2020/195037

### Non-Patent Document

Non-Patent Document 1: J. Ferment. Bioeng. (1997) Vol. 84, p. 319

### SUMMARY OF INVENTION

### Technical Problem

As it has recently become possible to produce almost all rare sugars, the shift from the laboratory-level mass production of rare sugar D-allose whose physiological activity has been clarified to the market-scale mass production of it, which leads to industrialization, is a new technical problem for the present inventors. For achieving highly-efficient mass production of rare sugars, a technical problem has been to select an enzyme having highly heat-resistant activity and a wide range of an optimum pH. Further, it becomes necessary to select an enzyme having not only a higher optimum reaction temperature and higher heat resistance but also an optimum pH on a more acidic side at which alkali isomerization of a rare sugar is unlikely to occur.

According to the conventional technology, as described above, rare sugar D-allose is produced from pure rare sugar D-allulose as a raw material by using a known L-rhamnose isomerase (EC 5.3.1.14). A L-rhamnose isomerase is an enzyme that catalyzes the isomerization reaction from L-rhamnose to L-rhamnurose and can also catalyzes the isomerization from L-rhamnurose to L-rhamnose. It is also known to act on the isomerization between D-allose and D-allulose. An isomerization enzyme is named based on a substrate showing the highest activity so that enzymes named as L-rhamnose isomerase have various substrate specificities.

Some known enzymes produce D-altrose, which is one of aldoses, as a byproduct during the production of D-allose and the presence of byproduct D-altrose becomes a cause for reducing the yield of D-allose in a D-allose purification step. Therefore, the purity of rare sugar D-allose in mass production becomes one of the problems.

Object of the present invention are to provide a novel L-rhamnose isomerase that is derived from a microorganism approved for use in food production and considered to have no toxicity, has high heat resistance, has an optimum pH on a more acidic side, and capable of isomerizing D-allulose into D-allose in a high yield; a microorganism having the enzyme, and a production method using the enzyme.

### Solution to Problem

Paying attention to the kind of bacteria listed as an approved food not only in Japan but also in Europe and the United States and having no toxicity, the present inventors collected soils of various places, separated microorganisms therefrom, and continued to search for a microorganism having L-rhamnose isomerase activity.

As a result, they have found, from a number of isolated strains, a microorganism that belongs to the genus Enterobacter and produces a novel L-rhamnose isomerase. Enterobacter roggenkampii, this microorganism belonging to the genus Enterobacter, produces a novel L-rhamnose isomerase having high heat resistance and having an optimum pH on a more acidic side.

The novel L-rhamnose isomerase derived from the aforesaid microorganism catalyzes the isomerization reaction between an aldose and a ketose corresponding thereto, recognizes and reacts with the CHO group at C1 and the OH group at C2 of the aldose to convert the CHO group at C1 into an OH group and the OH group at C2 into a CO group, or recognizes and reacts with the OH group at C1 and the CO group at C2 of the ketose to convert the OH group at C1 into a CHO group and the CO group at C2 into an OH group; and thus has isomerase activity between an aldose and a ketose. During production of D-allose with a D-allulose as a substrate, it does not produce a byproduct D-altrose so that it is suited for the production of D-allose.

The present invention therefore relates to an L-rhamnose isomerase described in the following (1) to (4).
(1) A L-rhamnose isomerase derived from a microorganism belonging to the genus Enterobacter, having a subunit molecular mass of 47 kDa as measured by SDS-PAGE, and having the following substrate specificities (A) and (B):
   (A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
   (B) having reactivity with L-rhamnose, L-lyxose, L-mannose, D-allose, D-ribose, and L-talose.
(2) The L-rhamnose isomerase as described above in (1), having the following physicochemical properties (C) and (D):
   (C) having an optimum reaction pH of 8.
   (D) having an optimum reaction temperature of 80°C.
(3) The L-rhamnose isomerase as described above in (1) or (2), wherein the microorganism belonging to the genus Enterobacter is Enterobacter roggenkampii.
(4) The L-rhamnose isomerase as described in any of (1) to (3), wherein the microorganism belonging to the genus Enterobacter is Enterobacter roggenkampii NrT7-1 internationally deposited at NITE Patent Microorganisms Depositary under accession number of NITE BP-03309.

The present invention also relates to a protein as described in the following (5), a DNA, recombinant vector, or a transformed host cell as described in the following (6) to (9), and a microorganism as described in the following (10).
(5) A protein described in the following (a) or (b):
   (a) a protein having an amino acid sequence represented by SEQ ID NO: 1.
   (b) a protein having an amino acid sequence having 80% or more identity with the amino acid sequence represented by SEQ ID NO: 1 and having L-rhamnose isomerase activity described in the following (A) and (B):
      (A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
      (B) having reactivity with L-rhamnose, L-lyxose, L-mannose, D-allose, D-ribose, and L-talose.
(6) A DNA encoding the protein as described above in (5).
(7) a DNA described in the following (a) or (b).
   (a) a DNA having a base sequence represented by SEQ ID NO: 2.
   (b) a DNA having a base sequence having 80% or more identity with the base sequence represented by SEQ ID NO: 2 and encoding a protein having L-rhamnose isomerase activity described in the following (A) and (B):
      (A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
      (B) having reactivity with L-rhamnose, L-lyxose, L-mannose, D-allose, D-ribose, and L-talose.
(8) A recombinant vector containing the DNA as described above in (6) or (7).
(9) A transformed host cell obtained by transformation with the recombinant vector as described above in (8).
(10) Enterobacter roggenkampii NrT7-1 which produces the L-rhamnose isomerase as described above in any of (1) to (4) and is internationally deposited at Patent Microorganisms Depositary under accession number of NITE BP-03309.

The present invention further relates to an immobilized protein as described in the following (11) to (13).
(11) An immobilized protein in which the L-rhamnose isomerase as described above in any of (1) to (4) or the protein as described above in (5) is immobilized on a carrier.
(12) An immobilized protein in which the L-rhamnose isomerase as described above in any of (1) to (4) is immobilized on a carrier in the form of a crude enzyme present in a disrupted cell or the protein as described above in (5) is immobilized on a carrier in the form of a crude protein present in a disrupted cell of a transformed host cell.
(13) The immobilized protein as described above in (11) or (12), wherein the carrier is an ion exchange resin or a synthetic adsorbent.

The present invention further relates to a method of producing an L-rhamnose isomerase or a method of producing a ketose or aldose as described in the following (14) to (16).
(14) A method of producing an L-rhamnose isomerase, comprising culturing the microorganism belonging to the genus Enterobacter producing the L-rhamnose isomerase as described above in any of (1) to (4) or the transformed host cell as claimed in (9), in a culture medium, to accumulate the L-rhamnose isomerase in a microorganism cell and collecting the accumulated enzyme.
(15) The method of producing an L-rhamnose isomerase as described above in (14), wherein the culture medium is an L-rhamnose-added inorganic salt one.
(16) A method of producing a ketose or an aldose, including causing the L-rhamnose isomerase as described in any of (1) to (4), the protein as described above in (5), or the immobilized protein as described above in any of (11) to (13) to act on a solution containing at least one selected from aldoses and ketoses to produce a corresponding ketose or aldose and collecting the resulting ketose or aldose.

### Advantageous Effects of Invention

The L-rhamnose isomerase of the present invention is characterized by that it has particularly high heat resistance and has an optimum pH on a more acidic side, compared with a conventional L-rhamnose isomerase derived from a microorganism.

For example, the conventional L-rhamnose isomerase derived from Erwinia billingiae GuaL218-3 and L-rhamnose isomerase derived from Arthrobacter globiformis M30 have optimum temperatures of 70°C and 50°C in a 10-min reaction, respectively, showing that the L-rhamnose isomerase of the present invention with an optimum temperature of 80°C is superior to them.

The L-rhamnose isomerase of the present invention has an optimum pH of pH 8.0 (Tris-HCl buffer) in a 10-min reaction. It has an optimum pH on a more acidic side compared with the L-rhamnose isomerase derived from GuaL218-3 having an optimum pH of 9.0 (Glycine-NaOH buffer) and the L-rhamnose isomerase derived from M30 having an optimum pH of 10.0 (Glycine-NaOH buffer) so that it is less likely to cause alkali isomerization and is therefore advantageous for D-allose production.

Further, when the substrate is D-allulose, the enzyme of the present invention converts it into only D-allose and does not produce a byproduct D-altrose, resulting in an increase in the production yield.

### BRIEF DESCRIPTION OF DRAWINGS

Fig. 1 shows the results of SDS-PAGE of the present enzyme.
Fig. 2 shows the optimum pH of the present enzyme.
Fig. 3 shows the pH stability of the present enzyme after 24 hours.
Fig. 4 shows the optimum temperature of the present enzyme.
Fig. 5 shows the temperature stability of the present enzyme after 10-min heat insulation (60 °C).
Fig. 6 shows the substrate specificity of the present enzyme.
Fig. 7 shows the influence of a metal ion on the present enzyme.
Fig. 8 shows the HPLC analysis results of the isomerization reaction from D-allulose to D-allose by using a recombinant enzyme.
Fig. 9 shows the optimum pH of a recombinant enzyme.
Fig. 10 shows the pH stability of the recombinant enzyme after 24 hours.
Fig. 11 shows the optimum temperature of the recombinant enzyme.
Fig. 12 shows the temperature stability of the recombinant enzyme after 10-min heat insulation (60°C).
Fig. 13 shows the substrate specificity of the recombinant enzyme.
Fig. 14 shows the influence of a metal ion on the recombinant enzyme.

### DESCRIPTION OF EMBODIMENTS

The present invention relates to an L-rhamnose isomerase which can be isolated from a microorganism belonging to the genus Enterobacter. It has characteristic properties such as high heat resistance and an optimum pH on a more acidic side.

The L-rhamnose isomerase has isomerase activity in which it recognizes and reacts with the CHO group at C1 and the OH group at C2 of an aldose and converts the CHO group at C1 into an OH group and the OH group at C2 into a CO group to yield a ketose; or recognizes and reacts with the OH group at C1 and the CO group at C2 of a ketose and converts the OH group at C1 into a CHO group and the CO group at C2 into an OH group to yield an aldose.

The term "ketose" as used herein means a ketohexose which is a hexose with a ketose structure or a ketopentose which is a pentose with a ketose structure. The ketohexose includes allulose (another name: psicose), sorbose, tagatose, and fructose, while the ketopentose includes ribulose and xylulose.

The term "aldose" as used herein means an aldohexose which is a hexose with an aldose structure or an aldopentose which is a pentose with an aldose structure. The aldohexose includes glucose, allose, altrose, gulose, idose, talose, galactose, and mannose, while the aldopentose includes ribose, arabinose, xylose, and lyxose. The term "D-" or "L-" means the D-form or L-form of the aforesaid ones.

The L-rhamnose isomerase of the present invention acts on L-rhamnose, L-lyxose, L-mannose, D-ribose, L-talose, and D-allose, can catalyze the conversion between L-rhamnose and L-rhamnurose, between L-lyxose and L-xylose, between L-mannose and L-fructose, between D-ribose and D-ribulose, between L-talose and L-tagatose, and between D-allose and D-allulose, and thus has a wide substrate specificity.

The L-rhamnose isomerase of the present invention is prepared by culturing a microorganism belonging to the genus Enterobacter and having L-rhamnose isomerase production ability; and isolating the L-rhamnose isomerase from cells grown in a culture medium. As the microorganism belonging to the genus Enterobacter, Enterobacter roggenkampii NrT7-1 and variants thereof, for example, can be used advantageously. In filing the present application, the NrT7-1 strain was internationally deposited at National Institute of Technology and Evaluation, Patent Microorganisms Depositary at 2-5-8 Kazusakamatari, Kisarazu-shi, Chiba, Japan on November 2, 2020 and accepted under reference number NITE ABP-03309. Then, the NrT7-1 strain was internationally deposited on November 30, 2020 under accession number NITE BP-03309 duly under the Budapest Treaty.

With respect to the L-rhamnose isomerase of the present invention, after the aeration culture of an L-rhamnose isomerase-producing microorganism belonging to the genus Enterobacter in an L-rhamnose-added inorganic salt medium, the cells are collected from the culture solution by centrifugal separation. The cells thus collected are washed with a 10 mM Tris-HCl buffer (pH 7.5) and suspended in 10 mL of a 10 mM Tris-HCl buffer (pH 7.5). The resulting cell suspension is subjected to cell disruption with an ultrasonic homogenizer while cooling in ice water. The homogenate thus obtained is centrifuged and the supernatant thus obtained is used as a crude enzyme solution.

The activity of the L-rhamnose isomerase in the crude enzyme solution before purification can be found by using D-allulose as a substrate and measuring the production amount of D-allose.

The enzyme activity in conversion from D-allose into D-allulose, which is a reverse reaction, is also measured under similar conditions. These conversion reactions are usually performed under the following conditions. The substrate concentration is 1 to 60% (w/v), desirably about 5 to 50% (w/v); the reaction temperature is 40 to 90°C, desirably about 60 to 90°C; and the reaction pH is 6 to 10, desirably about 6 to 9. The reaction time can be selected as needed and in a batch reaction, the reaction time is usually selected from a range of 4 to 20 hours.

The crude enzyme solution is purified by performing anion exchange chromatography, hydrophobic chromatography, and anion exchange chromatography successively to isolate a purified enzyme. In order to confirm the purification of the enzyme, it is subjected to SDS-PAGE (gel concentration: 12.5%) to find that it has a single band and in addition, the apparent molecular mass of it.

The L-rhamnose isomerase of the present invention purified as described above has a subunit molecular mass, by SDS-PAGE, of about 47 kDa and it is a metal enzyme whose activation level is controlled by a metal ion. The reaction with the substrate may be performed in the presence of a metal ion selected from the group consisting of manganese, cobalt, magnesium, silver, iron, copper, zinc, nickel, calcium, aluminum, and sodium at a concentration of 0.5 to 5 mM.

The L-rhamnose isomerase of the present invention has a predetermined amino acid sequence and examples of it include a protein having an amino acid sequence represented by SEQ ID NO: 1 and a protein having an amino acid sequence homologous thereto and maintaining L-rhamnose isomerase activity equal to that of the protein. The term "having an amino acid sequence homologous thereto" means that it has 80% or more, preferably 85% or more, more preferably 86, 87, 88, 89, 90, 91, 92, 93, or 94% or more, and still more preferably 95% or more identity with the amino acid sequence of SEQ ID NO: 1.

The identity (%) between two amino acid sequences or two nucleic acid sequences (base sequences) can be determined, for example, by the following procedure. First, two sequences are arranged to enable the optimal comparison. At this time, a gap is introduced into a first sequence to optimize the alignment with a second sequence. When a molecule (amino acid residue or nucleotide) at a certain position of the first sequence and a molecule at the corresponding position in the second sequence are the same, the respective molecules at these positions are regarded to be the same. The identity of these two sequences is a function (meaning that the identity (%) = the number of the same positions / the total number of the positions x 100) of the number of the same positions common in these two sequences. Preferably, the number and size of the gap required for the optimization of the alignment are taken into consideration.

The comparison between two sequences and determination of the identity of them can also be achieved using a mathematical algorithm. Specific examples of the mathematical algorithm usable for the comparison between sequences include, but not limited to, the algorithm described in Karlin and Altschul (1990) Proc. Natl. Acad. Sci. USA 87: 2264-68 and altered in Karlin and Altschul (1993) Proc. Natl. Acad. Sci. USA 90: 5873-7. Such an algorithm is included in the NBLAST program and XBLAST program (version 2.0) described in Altschul et al. (1990) J. Mol. Biol. 215: 403-10. A nucleotide sequence equivalent to the nucleic acid molecule of the present invention may be obtained by carrying out BLAST nucleotide searches, for example, with the NBLAST program, score of 100, and wordlength of 12.

The DNA of the present invention is a gene encoding the L-rhamnose isomerase activity of the present invention and has a predetermined base sequence. Examples of it include a DNA sequence encoding the amino acid sequence represented by SEQ ID NO: 1 and a DNA sequence having a base sequence represented by SEQ ID NO: 2 or a base sequence homologous to the base sequence represented by SEQ ID NO: 2 and encoding a protein maintaining the L-rhamnose isomerase activity equal to that of the protein of SEQ ID NO: 1.

The term "base sequence homologous to" means, for example, a base sequence having 80% or more, preferably 85% or more, more preferably 86, 87, 88, 89, 90, 91, 92, 93, or 94% or more, and still more preferably 95% or more identity with the base sequence of SEQ ID NO: 2.

The DNA of the present invention can be isolated using known techniques such as hybridization technique and PCR technique in combination. An L-rhamnose isomerase variant can be obtained by base substitution by site-directed mutagenesis. The site-directed mutagenesis can be carried out by an optional method such as inverse PCR method or annealing method (ed. by Muramatsu et al, "New Handbook of Genetic Engineering, 4th revised edition", Yodosha, p. 82-88).

The DNA of the present invention may be inserted in a suitable autonomously-replicable vector to obtain a recombinant vector. A recombinant vector comprises a DNA and an autonomously-replicable vector so that it can be obtained relatively easily by a conventional recombinant DNA technique when the DNA is available. An appropriate vector is selected, depending on the using purpose such as cloning or protein expression, or depending on a host cell. Examples of such a vector include plasmid vectors such as pBR322, pUC18, pUB110, pTZ4, pC194, pHV14, TRp7 YEp7, and pBS7 and phage vectors such as λgt·λC, λgt·λB, ρ11, ϕ1, and ϕ105.

The recombinant vector thus obtained can be introduced into a suitable host cell such as Escherichia coli, Bacillus subtilis, actinomycete, or a yeast. For the introduction, a known method such as calcium phosphate coprecipitation method, electroporation method, lipofection method, or microinjection method is used. The transformed host cells are obtained by a colony hybridization method or the like.

A method of producing the L-rhamnose isomerase and the protein having L-rhamnose isomerase activity according to the present invention is not particularly limited and a known method can be used. Described specifically, the L-rhamnose isomerase of the present invention can be produced by culturing, in a nutrition medium, the microorganism having L-rhamnose isomerase production ability according to the present invention or the host cells transformed by the DNA encoding the protein having L-rhamnose isomerase activity according to the present invention, and collecting the protein having L-rhamnose isomerase activity from the cultured product. As the culture method, a known method may be used and for example, either liquid culture or solid culture is usable.

After the cells are cultured as described above, the enzyme or protein of the present invention is purified and collected. The enzyme or protein can be purified and collected by a freely selected known method. For example, when they are collected from a culture solution, an insoluble matter is removed by subjecting the culture supernatant to filtration, centrifugal treatment, or the like, followed by separation and/or purification by using, in combination, concentration with an ultrafiltration membrane, salting-out such as ammonium sulfate precipitation, dialysis, and various chromatography techniques such as a technique using ion exchange resin, as needed. When they are collected from the cells inside, the cells are disrupted, for example, by lytic enzyme treatment or ultrasonic treatment, followed by separation and/or purification similar to those described above.

The L-rhamnose isomerase of the present invention, similar to another isomerase, catalyzes the isomerization reaction which does not require a coenzyme so that it can be used as an immobilized enzyme. An immobilized enzyme having high activity can be obtained by various immobilizing methods. Using an immobilized enzyme permits a continuous isomerization reaction of a large amount. The immobilized enzyme can be obtained utilizing a known immobilizing method, for example, a carrier binding method, a crosslinking method, a gel entrapment method, or a microcapsulation method. As the carrier, any known carrier may be used.

One mode of the method of immobilizing the L-rhamnose isomerase of the present invention is immobilization with a crude enzyme solution. The L-rhamnose isomerase can be immobilized by adding a crude enzyme solution containing the L-rhamnose isomerase, which solution is obtained by ultrasonically treating the cell suspension, to an ion exchange resin or the like and binding them at a low temperature.

In order to take out the L-rhamnose isomerase from the cells, disruption of their cell wall is required. As described above, there are disruption methods such as ultrasonic treatment and enzyme treatment with lysozyme. It has been revealed that the crude enzyme solution obtained by ultrasonic treatment contains a larger amount of the L-rhamnose isomerase having activity. As a carrier for immobilizing the enzyme from the crude enzyme solution, any known one is usable as an immobilizing carrier and ion exchange resins, sodium alginate, synthetic adsorbents, and the like are frequently used because of their convenience.

As a basic anion exchange resin, among the ion exchange resins, either a strongly basic anion exchange resin or a weakly basic anion exchange resin is usable. Examples of the strongly basic anion exchange resin include SA20A and PA418 (products of Mitsubishi Chemical) and examples of the weakly basic anion exchange resin include WA30 (product of Mitsubishi Chemical), and FPA54 and EPA95 (products of Organo). Examples of the synthetic adsorbent include XAD7HP (product of Organo).

When an immobilized enzyme is produced using the weakly basic anion exchange resin as a carrier, the L-rhamnose isomerase thus immobilized thereon can be eluted easily after reaction and an immobilized enzyme can therefore be regenerated very conveniently. This leads to improvement in production efficiency.

The L-rhamnose isomerase of the present invention can be produced by culturing, in a nutrient medium, the microorganism having L-rhamnose isomerase production ability or host cells transformed with the DNA encoding the protein having L-rhamnose isomerase activity, each according to the present invention; and collecting the protein having L-rhamnose isomerase activity from the cultured product. As the culturing method, a known method can be used and for example, either liquid culture or solid culture may be used.

After the cells are cultured in a medium, the resulting cultured product is purified and the L-rhamnose isomerase of the present invention is collected. Purification and collection of the protein may be performed by a freely selected known method. For example, when they are collected from the culture solution, an insoluble matter is removed by subjecting the culture supernatant to filtration, centrifugal treatment, or the like, followed by separation and purification by using, in combination, concentration with an ultrafiltration membrane, salting-out such as ammonium sulfate precipitation, dialysis, and various chromatography techniques such as a technique using an ion exchange resin, as needed. When they are collected from the cells, the cells are disrupted, for example, by lytic enzyme treatment or ultrasonic treatment, followed by separation and purification similar to those described above.

The purified L-rhamnose isomerase or immobilized enzyme thereof according to the present invention is caused to act on a solution containing at least one selected from aldoses and ketoses which will serve as a substrate to form a corresponding ketose or aldose and thus, they can be produced. The L-rhamnose isomerase of the present invention has a high substrate specificity to D-allose and compared with a conventional L-rhamnose isomerase, has an optimum temperature as high as 80°C and has an optimum pH on a more acidic side so that it is unlikely to cause alkali isomerization and is therefore advantageous for D-allose production.

### Examples

The present invention will be described in detail by experiments but the present invention is not limited by the following Examples.

### <Experiment 1: Origin and identification of strain>

The present inventors measured the activity of an L-rhamnose isomerase of many microorganisms which were isolated by screening, by inoculating them in a liquid medium to which L-rhamnose was added, carrying out shaking culture, and measuring the production amount of L-rhamnurose with L-rhamnose as a substrate.

Finding, in such a manner, the microorganism NrT7-1 strain as a strain having the highest activity, they revealed by phylogenetic analysis based on 16S rRNA gene base sequence homology that it belonged to the genus Enterobacter.

### Identification of strain

### (1) 16S rRNA gene base sequence homology

The 1-500 bp region of the 16s RNA gene was analyzed and 467 base pairs were specified.

### (2) Homology search

Homology search of the base sequence of the 16S rRNA gene of this strain with a known cell species considered as a standard strain by BLAST search (DNA Data Bank of Japan) was performed. The microorganism of the NrT7-1 strain was determined as Enterobacter roggenkampii from the name and homology value (%) of the strain having 99.5% or more homology to the base sequence number of 467 bp specified above.

The NrT7-1 strain was internationally deposited at National Institute of Technology and Evaluation, Patent Microorganisms Depositary on November 2, 2020 and was internationally deposited under accession number NITE BP-03309.

### <Experiment 2: Culture of Enterobacter roggenkampii NrT7-1 (accession number NITE BP-03309) strain>

In an inorganic salt liquid medium using 1.0% L-rhamnose as a carbon source and ammonium sulfate as a nitrogen source, a 1% (v/v) seed culture solution of the Enterobacter roggenkampii NrT7-1 (accession number NITE BP-03309) strain was aseptically added and cultured at 30°C for 24 hours while stirring under aeration.

### <Experiment 3: Preparation of crude enzyme>

The cells were collected from the culture solution of the NrT7-1 strain by centrifugal separation. After the cells thus collected were each washed with a 10 mM Glycine-NaOH buffer (pH 9.0) and then, suspended in a 10 mL Glycine-NaOH buffer (pH 9.0), the resulting cell suspension was disrupted with an ultrasonic homogenizer (Emerson Japan) while cooling in ice water. The disrupted products were centrifuged at 12,000 rpm for 20 minutes and the resulting supernatants were provided as crude enzymes, respectively.

### <Experiment 4: Purification of enzyme>

1. Purification by ion exchange chromatography and hydrophobic chromatography
   The crude enzyme solution was purified by ion chromatography. The column used therefor was HiTrapQ HP equilibrated with a buffer (20 mM Tris-HCl, pH 7.5) and the crude enzyme solution was fractionated using an AKTA system with a concentration gradient of 0% to 100% of 1M NaCl at a flow rate of 5 mL/min to obtain 5 mL fractions. The fractions in which enzyme activity was detected were collected and enzymes purified by ion exchange chromatographic separation were obtained.
2. The enzymes purified by ion exchange chromatographic separation were purified further by hydrophobic chromatography. The column used therefor was HiTrap PHENYL and ammonium sulfate was added to and dissolved in the enzyme solution to give a 1M solution. The resulting solution was eluted while decreasing the concentration of 2M ammonium sulfate from 100% to 0% and fractionated into 5 mL fractions. The fractions in which enzyme activity was detected were dialyzed to remove ammonium sulfate and enzymes purified by hydrophobic chromatographic separation were obtained.
3. The enzymes purified by hydrophobic chromatography were purified further by anionic exchange chromatography. The column used therefore was Resource-Q. After the enzyme solution was poured, it was fractionated with a concentration gradient of 0% to 100% of 1M NaCl at a flow rate of 6 mL/min to obtain 6 mL fractions. The fractions in which enzyme activity was detected were collected and thus, enzymes purified by ion exchange chromatographic separation were obtained.

### 3. Polyacrylamide gel electrophoresis

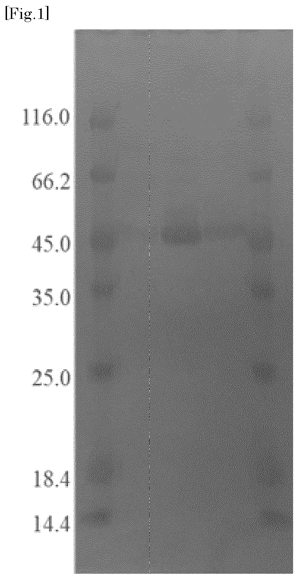

SDS-PAGE (gel concentration: 12.5%) was performed by a conventional method and the purity of the purified enzyme was confirmed. The results are shown in Fig. 1. According to the results, a single band (central lane) was recognized at about 47 kDa, showing that the enzyme was purified purely. It has been revealed that the present enzyme thus purified had a subunit molecular mass of about 47 kDa on SDS-PAGE.

### <Experiment 5: Measurement of physicochemical properties of enzyme>

The activity of the L-rhamnose isomerase, which was a purified enzyme, was measured by making the following experiment to conduct an enzyme reaction. The enzyme reaction was conducted for 10 minutes by using 5 mM L-rhamnose as a substrate under the following conditions. After the reaction, the reaction was stopped by adding 50 µL of 10% trichloroacetic acid solution to the reaction mixture. The L-rhamnurose thus generated was measured using the cysteine carbazole sulfuric acid method.

### 1. Optimum Reaction pH

In measurement, 5 mM L-rhamnose was used as a substrate. Various buffers of pH 4 to 11 were used and the reaction was performed at 30°C for 10 minutes. The optimum reaction pH was found by measuring the resulting L-rhamnurose by the cysteine carbazole sulfuric acid method.

The reaction conditions are shown in Table 1. The buffers used are shown in Table 2.

**[Table 1]**

| Reaction conditions | |
|---|---|
| Enzyme solution | |
| L-rhamnose | Final concentration: 5 mM |
| Manganese chloride | Final concentration: 1 mM |
| Buffers (pH 4.0 to 11.0) | Final concentration: 50 mM |
| Reaction temperature | 30°C |
| Reaction time | 10 minutes |

**[Table 2]**

| Buffer | |
|---|---|
| Acetate | pH 4.0, pH 5.0, pH 6.0 |
| MES | pH 6.0, pH 7.0 |
| MOPS | pH 7.0, pH 7.5 |
| Tris-HCl | pH 7.5, pH 8.0, pH 9.0 |
| Glycine-NaOH | pH 9.0, pH 10.0, pH 11.0 |

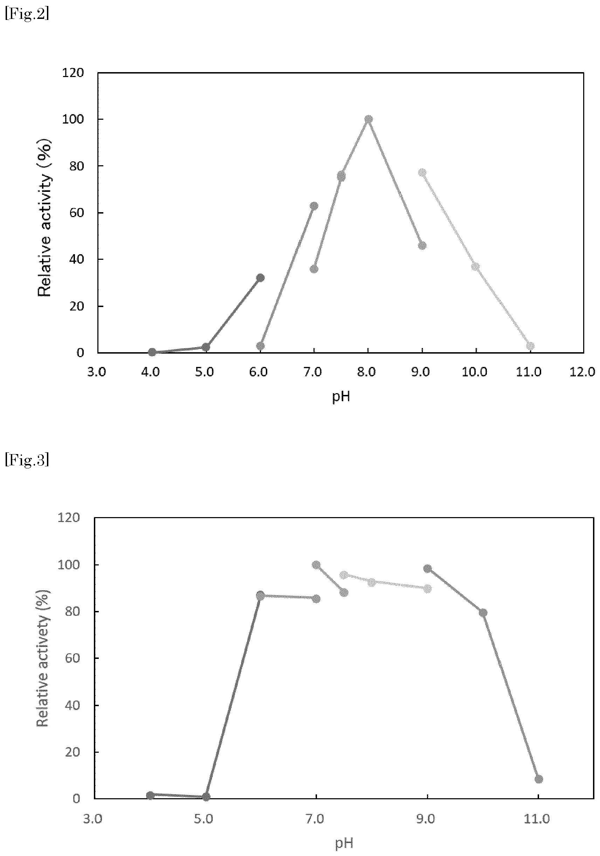

The results are shown in Fig. 2. The present enzyme has an optimum reaction pH of 8 and shows high activity on a more acidic side than a conventional enzyme.

### 2. PH stability

Next, four buffers of pH 4 to pH 11 shown in Table 2 were used and the residual activity after being retained at 30°C for 24 hours in each buffer is shown in Fig. 3. The present enzyme was stable at pH 6 to 10.

### 3. Optimum reaction temperature

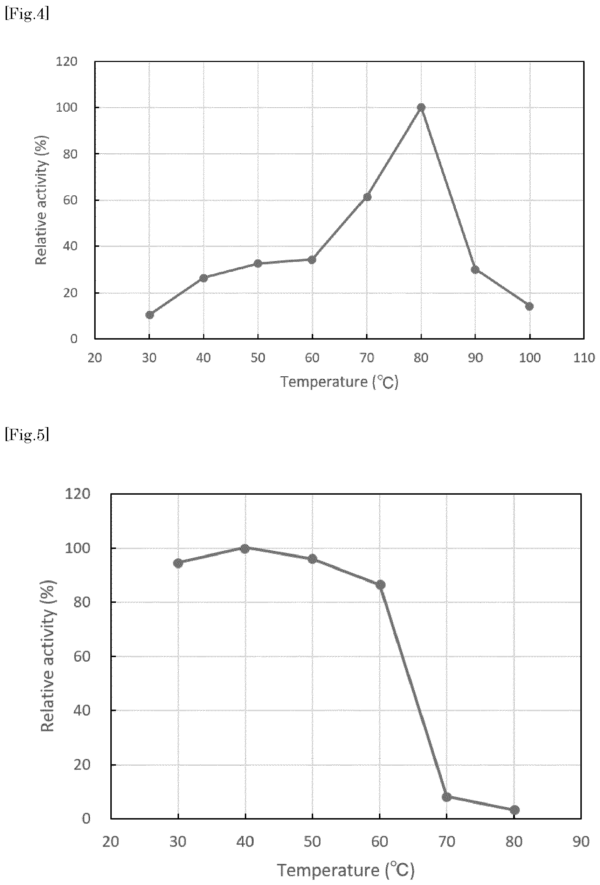

The pH was adjusted to 8 with a Tris-HCl buffer and the reaction was performed at various temperatures in a range of 30 to 100°C to find an optimum temperature. The reaction conditions are shown in Table 3 and the results are shown in Fig. 4. The present enzyme was found to have enzyme activity at each temperature and the optimum temperature was found to be 80°C.

**[Table 3]**

| Reaction conditions | |
|---|---|
| Enzyme solution | |
| L-Rhamnose | Final concentration: 5 mM |
| Manganese chloride | Final concentration: 1 mM |
| Tris-HCl buffer (pH 8.0) | Final concentration: 50 mM |
| Reaction temperature | 30 to 100°C |
| Reaction time | 10 minutes |

### 4. Thermal stability

The residual activity of the present enzyme after it was kept at each temperature for 10 minutes under the reaction conditions (10 minutes) shown in Table 3 under which the optimum temperature was found in above 3 is shown in Fig. 5. With respect to the temperature stability after the present enzyme was kept for 10 minutes at each temperature, a decrease in the relative activity of it at 60°C is less than 20%, revealing that it is a more stable enzyme at a high temperature.

### 5. Substrate specificity of L-rhamnose isomerase

The substrate specificity of the present enzyme to L-rhamnose and five aldoses (L-mannose, D-allose, L-talose, L-lyxose, and D-ribose) was studied. The enzyme reaction composition was similar to that shown in the reaction conditions of Table 3. The final concentration of each substrate was adjusted to 5 mM; manganese chloride was added as a metal salt to give a final concentration of 1 mM; and an enzyme solution (final concentration: 50 mM, Tris-HCl buffer, pH 8.0) was reacted at 60°C for 10 minutes. Respective ketoses obtained by isomerization of the aldoses were measured by the analysis with HPLC.

Supposing that the isomerization activity of L-rhamnose is 100, the activity to each aldose is shown as relative activity.

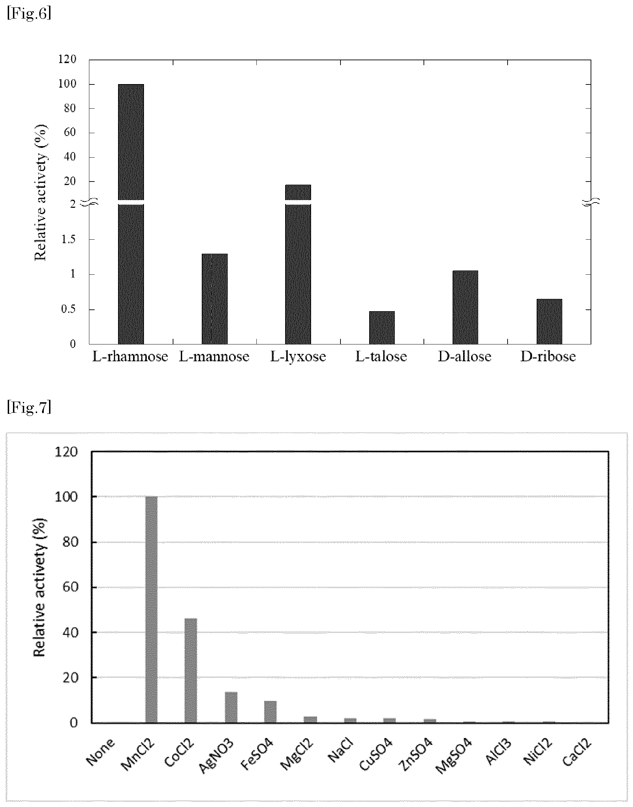

Activity when D-allose, L-Mannose, L-Talose, L-Lyxose, and D-Ribose are used as a substrate is shown as relative activity in Table 4 and Fig. 6.

**[Table 4]**

| Substrate | Relative activity (%) |
|---|---|
| L-Rhamnose | 100 |
| L-Lyxose | 17.2 |
| L-Mannose | 1.3 |
| D-Allose | 1.1 |
| D-Ribose | 0.7 |
| L-Talose | 0.5 |

The activity is the highest to L-rhamnose, followed by L-lyxose, L-mannose, D-allose, D-ribose, and L-talose. The present enzyme catalyzes the isomerization reaction between L-rhamnose and L-rhamnurose, L-lyxose and L- xylulose, L-mannose and L-fructose, D-allose and D-allulose, D-ribose and D-ribulose, and L-talose and L-tagatose.

### 6. Influence of a metal ion

Next, for the purpose of studying the influence of a metal ion on the activity of the present enzyme, a portion of the enzyme was dialyzed and enzyme activity thereof was measured. The dialysis was performed by placing an enzyme solution in a cellulose membrane, immersing the resulting membrane in a Tris-HCl buffer (pH 8.0) containing 20 mM EDTA, stirring the resulting buffer slowly over a period of 16 hours, and thereby removing the influence of an unintended metal ion. The enzyme activity of the apoenzyme thus obtained was measured by the cysteine carbazole method after reacting it in the presence (under the reaction conditions shown in Table 5) of various divalent and trivalent metal ions or sodium ions with a final concentration of 1 mM.

As a result, MnCl₂ and CoCl₂ markedly increased the activity of the present enzyme, showing the metal dependence of the present enzyme (Fig. 7).

**[Table 5]**

| (Reaction conditions when the influence of a metal ion is confirmed) | |
|---|---|
| Reaction conditions | |
| Buffer (Tris-HCl) | 350 µL |
| Substrate (50 mM L-rhamnose) | 50 µL |
| Dialyzed enzyme | 50 µL |
| Metal salt (10 mM) | 50 µL |
| Reaction temperature | 60°C |
| Reaction time | 10 minutes |

### <Experiment 6: Cloning of DNA encoding an enzyme and preparation of a recombinant vector containing the same and a transformed host cell>

A DNA encoding a protein having D-allose isomerase activity was cloned from the Enterobacter roggenkampii NrT7-1 strain, followed by the preparation of an autonomously replicable recombinant DNA, the determination of the base sequence of a DNA encoding the enzyme, and the preparation of a transformed microorganism.

### <Experiment 6-1: Determination of all the base sequences of a chromosomal DNA>

Different from the existing similar gene enzymes, an enzyme gene of the present microorganism having D-allose isomerase activity could not be isolated using a PCR amplification method or the existing protein data base. Then the whole genome sequence of the Enterobacter roggenkampii NrT7-1 strain was determined and a data base of the protein groups encoded by all ORFs in the genome was constructed. The present inventors asked Ltd. Microgen Japan to use, as a test cell, the cultured cells of the Enterobacter roggenkampii NrT7-1 strain and perform the next-generation sequence analysis of them with PacBio-RSII/Sequel.

As a result, two contigs having a base pair number of 4,751,985 bp and 161,947 bp, respectively, were obtained. Since the total base pair number of the two contigs was about 4.9 Mbp and the contigs were each cyclized and connected, it was presumed that they covered all the genome sequences of the Enterobacter roggenkampii NrT7-1 strain.

### <Example 6-2: Construction of a protein data base>

Based on the resulting DNA sequence of about 4.9 Mb, 4,569 ORFs were deduced using the Prokka program and the amino acid sequence was deduced for each ORF. The 4,569 amino acid sequences were used as a protein data base of the Enterobacter roggenkampii NrT7-1 strain.

### <Experiment 6-3: Identification of a protein having D-allulose isomerase activity>

The aforesaid protein data base was registered in the protein identification system MASCOT server (Matrix Science) to identify the protein showing D-allulose isomerase activity. A 47kDa band found by SDS-PAGE in the paragraph [0043] was used as a test sample and after reduction treatment and alkylation treatment, a fragment digested with trypsin was subjected to LC-MS/MS analysis.

As a result, the amino acid sequence of the sample coincided with the underlined amino acid sequence in the amino acid sequence having Sequence 1 (SEQ ID NO: 1 of Sequencing List) having the following 419 amino acids and had totally 90% identity therewith, strongly suggesting that the protein consisted of the amino acid sequence represented by SEQ ID NO: 1 was L-rhamnose isomerase of the Enterobacter roggenkampii NrT7-1 strain.

### [Sequence 1]

MTTQLEQAWDLAKQRFAAVGVDVEEALRQLDRLPVSMHCWQGDDVAGFENPGGSLT
GGIQATGNYPGKARNATELRADLELTLSLIPGPKRLNLHAIYLESDEPVARNEIKPEHFK
NWVEWAKANKLGLDFNPSCFSHPLSADGFTLAHADDEIRQFWIDHVKASRRVSAYFGE
QLGTPSVMNIWIPDGMKDITVDRLAPRQRLLAALDEAISEKLDPAHHIDAVESKLFGIGA
ESYTVGSNEFYMGYATSRQTALCLDAGHFHPTEVISDKISAAMLYVPRLLLHVSRPVRW
DSDHVVLLDDETQAIASEIIRHDLFDRVHIGLDFFDASINRIAAWVIGTRNMKKALLRAL
LEPTAELKKLEANGDYTARLALLEEQKSLPWQAVWEMYCQRNDAPAGSQWLDNVRA
YEEDVLSQRG

(Sequence 1: An amino acid sequence identified using the protein data base of the Enterobacter roggenkampii NrT7-1 strain. The underlined amino acid sequence is an amino acid sequence that agreed with the peak obtained by LC-MS/MS analysis.)

### < Experiment 6-4: Isolation of an enzyme gene having D-allulose isomerase activity>

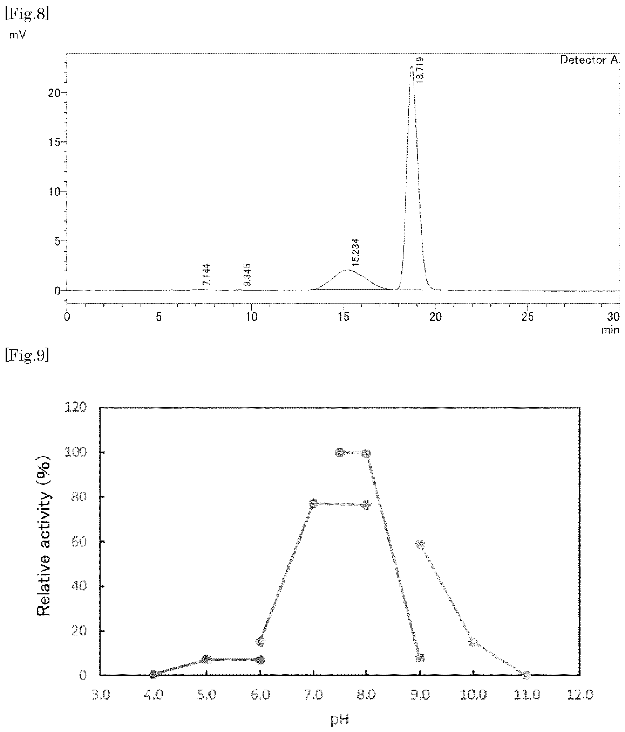

The DNA sequence (SEQ ID NO: 2 in Sequence Listing) of Sequence 2 of the gene identified from the amino acid sequence was synthesized, incorporated in a pQE60 vector (Qiagen), and used for the transformation of an Escherichia coli for expression. An inducible enzyme was confirmed using the thus-constructed Escherichia coli expression system. With the recombinant enzyme thus induced, 60% (w/v) D-allulose used as a substrate was reacted at 30°C for 12 hours and the D-allulose isomerase activity was confirmed by HPLC. As a result, the present recombinant enzyme catalyzed the isomerization reaction from D-allulose to D-allose and in addition, did not produce D-altrose, an undesired byproduct. The HPLC analysis results are shown in Fig. 8. The peak at 15.234 minutes indicates D-allose and the peak at 18,719 minutes indicates D-allulose.

### [Sequence 2]

ATGACCACTCAATTAGAACAAGCCTGGGACCTGGCTAAACAGCGTTTCGCCGCCGTC
GGCGTGGATGTCGAAGAGGCCCTGCGCCAGCTCGACCGTCTGCCCGTCTCTATGCAC
TGCTGGCAGGGCGATGACGTCGCCGGTTTCGAGAACCCGGGCGGTTCCCTGACGGG
TGGTATTCAGGCCACGGGTAATTATCCGGGCAAAGCGCGTAACGCGACCGAGCTGC
GCGCCGATCTGGAGCTGACCCTGAGCCTGATCCCGGGGCCAAAGCGCCTGAACCTG
CACGCGATTTACCTCGAGTCCGACGAGCCGGTGGCGCGCAACGAAATCAAACCGGA
GCATTTTAAGAACTGGGTGGAGTGGGCAAAAGCCAATAAGCTGGGCCTGGATTTCA
ACCCATCCTGCTTCTCGCATCCGCTGAGCGCGGACGGTTTTACCCTCGCCCACGCGG
ACGATGAAATCCGTCAGTTCTGGATCGACCACGTGAAGGCCAGCCGCCGCGTGTCT
GCGTACTTTGGCGAGCAGCTCGGCACACCGTCGGTGATGAACATCTGGATCCCGGA
CGGCATGAAGGACATCACCGTCGACCGTCTTGCTCCGCGCCAGCGCCTGCTGGCCGC
GCTGGATGAGGCCATCAGCGAGAAGCTGGACCCGGCGCACCACATCGACGCCGTGG
AGAGCAAGCTGTTCGGCATTGGCGCCGAGAGCTACACCGTCGGCTCGAACGAGTTC
TACATGGGGTACGCCACCAGCCGCCAGACCGCGCTGTGCCTGGATGCCGGTCACTTC
CATCCGACCGAGGTGATCTCCGACAAAATCTCCGCTGCCATGCTCTACGTGCCGCGC
CTGCTGCTGCACGTCAGCCGTCCGGTGCGCTGGGACAGCGACCACGTAGTGCTGCTG
GATGACGAAACCCAGGCGATTGCGAGCGAAATCATCCGCCACGACCTCTTTGACCG
CGTCCACATCGGCCTCGACTTCTTCGATGCCTCCATCAACCGCATCGCGGCGTGGGT
TATCGGCACCCGCAACATGAAGAAAGCCCTGCTGCGCGCCCTGCTGGAGCCGACTG
CCGAACTGAAAAAGCTGGAAGCAAACGGTGACTACACCGCGCGTCTGGCGCTGCTG

GAAGAGCAGAAATCTCTGCCGTGGCAGGCGGTGTGGGAGATGTACTGCCAGCGTAA
CGACGCCCCGGCGGGCAGCCAGTGGCTGGACAACGTGCGGGCGTATGAGGAAGAC
GTGTTGAGTCAGCGCGGGTAA

### <Experiment 7: Measurement of physicochemical properties of a recombinant enzyme>

The physicochemical properties of the recombinant enzyme obtained in Experiment 6 were measured under the conditions similar to those in Experiment 5.

### 1. Optimum reaction pH

In measurement, 5 mM L-rhamnose was used as a substrate. Various buffers of pH 4 to 11 were used and the reaction was performed at 30°C for 10 minutes. The optimum reaction pH was found by measuring the resulting L-rhamnurose by the cysteine carbazole sulfuric acid method. The reaction conditions are shown in Table 1. The buffers used are shown in Table 6.

**[Table 6]**

| Buffer | |
|---|---|
| Acetate | pH 4.0, pH 5.0, pH 6.0 |
| Sodium phosphate | pH 6.0, pH 7.0, p H 8.0 |
| Tris-HCl | pH 7.5, pH 8.0, pH 9.0 |
| Glycine-NaOH | pH 9.0, pH 10.0, pH 11.0 |

The results are shown in Fig. 9. The optimum pH of the recombinant enzyme was 7.5 to 8.0.

### 2. pH stability

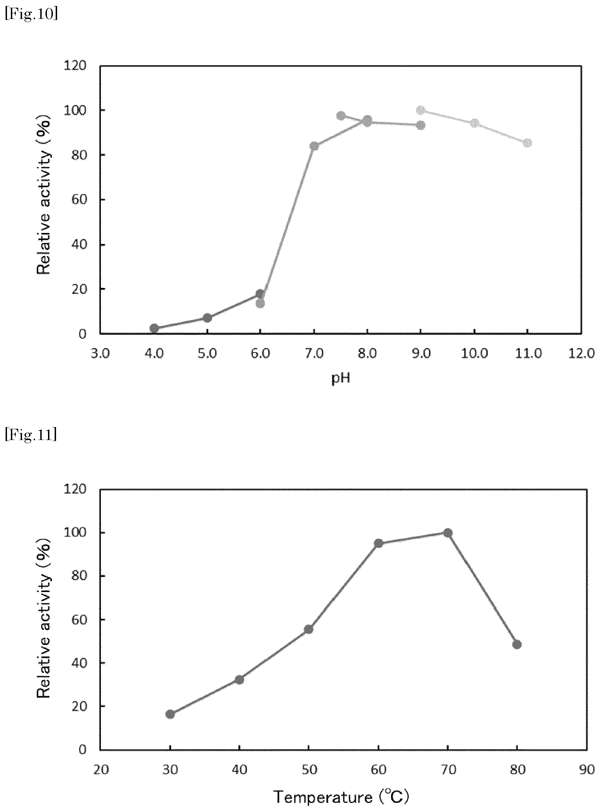

Four buffers of pH 4 to 11 in Table 6 were used and the residual activity after the enzyme was retained in each buffer at 30°C for 24 hours is shown in Fig. 10. The recombinant enzyme was stable at pH 7.0 to 11.0.

### 3. Optimum reaction temperature

After adjustment of the pH to 7.5 with a Tris-HCl buffer, the reaction was performed for 10 minutes at various temperatures in a range of from 30 to 80°C to find an optimum temperature. The results are shown in Fig. 11. The optimum temperature was found to be 70°C.

### 4. Thermal stability

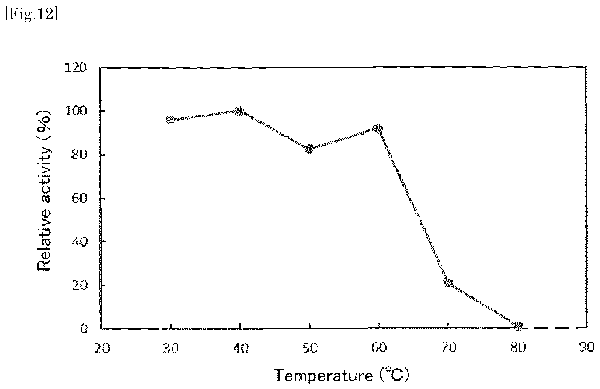

The residual activity of the recombinant enzyme after it was kept at each temperature for 10 minutes under the reaction conditions from which the optimum temperature was found in the above 3. is shown in Fig. 12. The temperature stability after it was kept for 10 minutes continued up to 60°C.

### 5. Substrate specificity

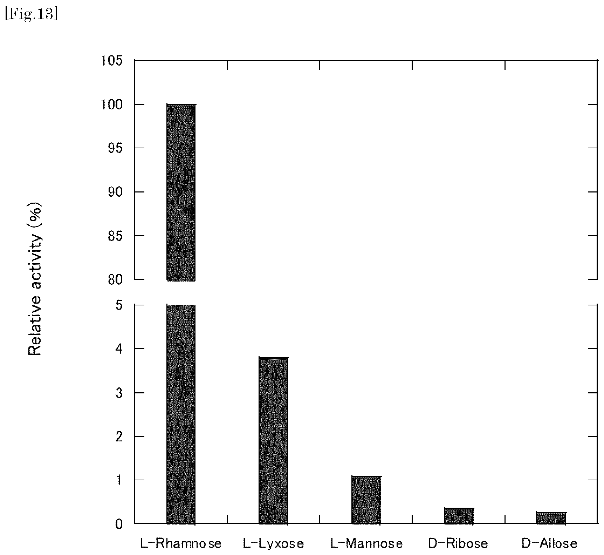

According to the enzyme reaction composition shown in Table 5, the final concentration of each substrate was adjusted to 5 mM; manganese chloride was added as a metal salt to give a final concentration of 1 mM; and an enzyme solution (final concentration: 50 mM, Tris-HCl buffer, pH 7.5) was reacted at 70°C for 10 minutes. Respective ketoses obtained by isomerization of aldoses were measured by the analysis with HPLC. Supposing that the isomerization activity of L-rhamnose is 100, the activity to each aldose is shown in Fig. 13 as relative activity.

The enzyme showed activity to L-rhamnose, L-Lyxose, L-Mannose, D-Ribose, and D-allose.

### 6. Influence of metal ion

For the purpose of studying the influence of a metal ion on the activity of the recombinant enzyme, a portion of the enzyme was dialyzed and enzyme activity thereof was measured. The dialysis was performed by placing an enzyme solution in a cellulose membrane, immersing the resulting membrane in a Tris-HCl buffer (pH 7.5) containing 20 mM EDTA, stirring the resulting buffer slowly over a period of 16 hours, and thereby removing the influence of an unintended metal ion. The enzyme activity of the apoenzyme thus obtained was measured by the cysteine carbazole method after reacting in the presence of various divalent and trivalent metal ions or sodium ions with a final concentration of 1 mM.

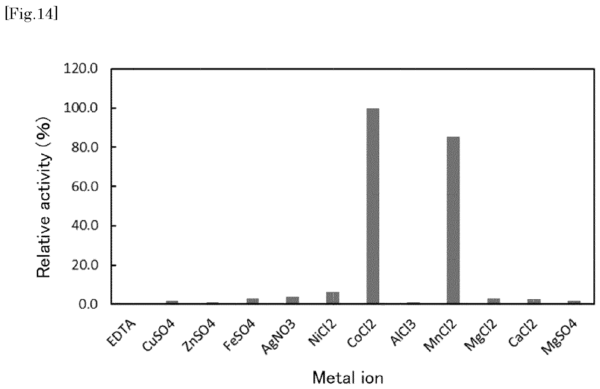

The results are shown in Fig. 14. Addition of CoCl₂ and MnCl₂ enhanced the activity.

### <Experiment 8: Comparison with an L-rhamnose isomerase derived from other strains (1)>

### 1. Influence of a metal ion on enzyme reaction

The influence of a metal ion on a reversible isomerization reaction between D-allulose and D-allose was compared between when the reaction was performed using the NrT7-1-derived recombinant crude enzyme thus obtained and when the reaction was performed using a GuaL218-3-derived recombinant crude enzyme or an AgM30-derived recombinant crude enzyme.

The results are shown in Table 7. When 2 mM CoCl₂ and MnCl₂ were used as a metal ion, the isomerization reaction was confirmed. Compared with the AgM30-derived recombinant crude enzyme which was a reference enzyme, and GuaL218-3-derived recombinant crude enzyme, , the NrT7-1-derived recombinant crude enzyme showed high activity.

**[Table 7]**

| | Substrate | Concentration of substrate (mM) | Metal ion | Product | Enzyme activity (U) | Comparison with activity of AgM30-derived LRhI |
|---|---|---|---|---|---|---|
| GuaL218-3-derived L-Rhamnose isomerase | D-allulose | 100 | 2 mM | D-allose | 13.1 | Activity 6.9-fold higher than Reference 1 |
| | D-allose | 100 | MnCl₂ | D-allulose | 52.1 | Activity 2.8-fold higher than Reference 2 |
| | D-allulose | 100 | 2 mM | D-allose | 10.3 | Activity 6.1-fold higher than Reference 3 |
| | D-allose | 100 | CoCl₂ | D-allulose | 73.4 | Activity 3.7-fold higher than Reference 4 |
| NrT7-1-derived L-Rhamnose isomerase | D-allulose | 100 | 2 mM | D-allose | 30.2 | Activity 15.9-fold higher than Reference 1 |
| | D-allose | 100 | MnCl₂ | D-allulose | 92.4 | Activity 4.9-fold higher than Reference 2 |
| | D-allulose | 100 | 2 mM | D-allose | 35.6 | Activity 20.9-fold higher than Reference 3 |
| | D-allose | 100 | CoCl₂ | D-allulose | 137.5 | Activity 6.9-fold higher than Reference 4 |
| AgM30-derived L-Rhamnose isomerase | D-allulose | 100 | 2 mM | D-allose | 1.9 | Reference 1 |
| | D-allose | 100 | MnCl₂ | D-allulose | 18.9 | Reference 2 |
| | D-allulose | 100 | 2 mM | D-allose | 1.7 | Reference 3 |
| | D-allose | 100 | CoCl₂ | D-allulose | 20.0 | Reference 4 |

### <Example 9: Influence of a metal ion at the time of culturing the present enzyme>

High activity was confirmed when 2 mM CoCl₂ or MnCl₂ was added as a metal ion at the time of reaction using the recombinant enzyme so that it was investigated whether or not the reaction was conducted without a metal ion at the time of reaction if the metal ion was added at the time of culturing. Using a crude enzyme obtained by culturing in a metal ion-containing medium, an enzyme reaction was performed with D-allulose as a substrate. Then as shown in Table 8, almost no activity was found when the metal ion was not added or MgCl₂ was added. On the other hand, the crude enzyme obtained by culturing in a MnCl₂-added or CoCl₂-added medium was found to have activity. In particular, the crude enzyme obtained by culturing in a MnCl₂-added medium showed the highest activity and in addition, had the highest residual activity when heat treated at 60°C for one hour.

**[Table 8]**

| | NrT7-1-derived LRhl | | | |
|---|---|---|---|---|
| Metal added to medium | None | MgCl₂ | CoCl₂ | MnCl₂ |
| Specific activity at 60°C (U/mL) | 0.34 | 0.2 | 1.07 | 2.52 |
| Specific activity after heat treatment at 60°C for one hour (U/mL) | Trace | Trace | 0.83 | 2.37 |
| Residual activity after heat treatment at 60°C for one hour (%) | - | - | 77.6 | 94.1 |

### <Experiment 10: Comparison with L-rhamnose isomerase derived from another strain (2)>

### 2. Optimum temperature and heat resistance (T70/T50)

The optimum temperature of the NrT7-1-derived recombinant crude enzyme obtained in the present study and that of the GuaL218-3-derived recombinant crude enzyme described in the previous patent were investigated.

As a result, the optimum temperature of the NrT7-1-derived recombinant crude enzyme was 70°C, higher than the optimum temperature (60°C) of the GuaL 218-3-derived recombinant crude enzyme. In addition, a ratio (T70/T50) of the activity (T70) at 70°C, which was an index of heat resistance, to the activity (T50) at 50°C was higher in the NrT7-1-derived recombinant crude enzyme than in the GuaL 218-3-derived recombinant crude enzyme.

**[Table 9]**

| | | GuaL 218-3-derived L-RhI | NrT7-1-derived L-RhI |
|---|---|---|---|
| Activity per 100 mL of medium (U) | Reaction at 50°C | 49.60 | 57.40 |
| | Reaction at 60°C | 75.54 | 71.31 |
| | Reaction at 70°C | 57.78 | 74.71 |
| | Reaction at 80°C | 5.78 | 32.18 |
| T70/T50 | | 1.16 | 1.30 |
| Residual activity after kept at 60°C for one hour (%) | 103.4 | 101.9 | |

### Industrial Applicability

The L-rhamnose isomerase of the present invention is characterized by that it has particularly high heat resistance compared with a conventional microorganism-derived L-rhamnose isomerase. For example, a conventional L-rhamnose isomerase derived from Erwinia billingiae has an optimum temperature of 70°C but the enzyme of the present invention has an optimum temperature as high as 80°C and is therefore suited for use in industrial production.

The L-rhamnose isomerase of the present invention has an optimum pH of 8.0 (Tris-HCl buffer) and thus has an optimum pH on a more acidic side than the GuaL218-3-derived L-rhamnose isomerase having an optimum pH of 9.0 (Glycine-NaOH buffer). This means that the enzyme of the present invention hardly causes alkali isomerization and is advantageous for D-allose production.

Accordingly, the L-rhamnose isomerase of the present invention and establishment of the production method thereof have a significantly large industrial meaning not only in the sugar production industry but also in the food, cosmetic, and pharmaceutical industries related thereto.

## Claims

1. An L-rhamnose isomerase derived from a microorganism belonging to the genus Enterobacter, having a subunit molecular mass of 47 kDa as measured by SDS-PAGE, and having the following substrate specificities (A) and (B):
(A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
(B) having reactivity with L-rhamnose, L-lyxose, L-mannose, D-allose, D-ribose, and L-talose.

2. The L-rhamnose isomerase according to Claim 1, having the following physicochemical properties (C) and (D):
(C) having an optimum reaction pH of 8.
(D) having an optimum reaction temperature of 80°C.

3. The L-rhamnose isomerase according to Claim 1 or 2, wherein the microorganism belonging to the genus Enterobacter is Enterobacter roggenkampii.

4. The L-rhamnose isomerase according to any of Claims 1 to 3, wherein the microorganism belonging to the genus Enterobacter is Enterobacter roggenkampii NrT7-1 internationally deposited at NITE Patent Microorganisms Depositary under accession number of NITE BP-03309.

5. A protein described in the following (a) or (b):
(a) a protein having an amino acid sequence represented by SEQ ID NO: 1.
(b) a protein having an amino acid sequence having 80% or more identity with the amino acid sequence represented by SEQ ID NO: 1 and having L-rhamnose isomerase activity described in the following (A) and (B):
(A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
(B) having reactivity with L-rhamnose, L-lyxose, L-mannose, D-allose, D-ribose, and L-talose.

6. A DNA encoding the protein as claimed in Claim 5.

7. A DNA described in the following (a) or (b).
(a) a DNA having a base sequence represented by SEQ ID NO: 2.
(b) a DNA having a base sequence having 80% or more identity with the base sequence represented by SEQ ID NO: 2 and encoding a protein having L-rhamnose isomerase activity described in the following (A) and (B):
(A) having isomerase activity that recognizes and reacts with a CHO group at C1 and an OH group at C2 of an aldose to convert the CHO group at C1 into an OH group and the OH group at C2 into a CO group, or recognizes and reacts with an OH group at C1 and a CO group at C2 of a ketose to convert the OH group at C1 into a CHO group and the CO group at C2 into an OH group.
(B) having reactivity with L-rhamnose, L-lyxose, L-mannose, D-allose, D-ribose, and L-talose.

8. A recombinant vector containing the DNA as claimed in Claim 6 or 7.

9. A transformant host cell obtained by transformation with the recombinant vector as claimed in Claim 8.

10. Enterobacter roggenkampii NrT7-1 which produces the L-rhamnose isomerase as claimed in any of Claims 1 to 4 and is internationally deposited at Patent Microorganisms Depositary under accession number of NITE BP-03309.

11. An immobilized protein in which the L-rhamnose isomerase as claimed in any of Claims 1 to 4 or the protein as claimed in Claim 5 is immobilized on a carrier.

12. An immobilized protein in which the L-rhamnose isomerase as claimed in any of Claims 1 to 4 is immobilized on a carrier in the form of a crude enzyme present in a disrupted cell or the protein as claimed in Claim 5 is immobilized on a carrier in the form of a crude protein present in a disrupted cell of a transformant host cell.

13. The immobilized protein according to Claim 11 or 12, wherein the carrier is an ion exchange resin or a synthetic adsorbent.

14. A method of producing an L-rhamnose isomerase, comprising culturing the microorganism belonging to the genus Enterobacter producing the L-rhamnose isomerase as described above in any of Claims 1 to 4 or the transformed host cell as claimed in Claim 9, in a culture medium, to accumulate the L-rhamnose isomerase in a microorganism cell and collecting the accumulated enzyme.

15. The method of producing an L-rhamnose isomerase according to Claim 14, wherein the culture medium is an L-rhamnose-added inorganic salt medium.

16. A method of producing a ketose or an aldose, comprising causing the L-rhamnose isomerase as claimed in any of Claims 1 to 4, the protein as claimed in Claim 5, or the immobilized protein as claimed in any of Claims 11 to 13 to act on a solution containing at least one selected from aldoses and ketoses to produce a corresponding ketose or aldose and collecting the resulting ketose or aldose.
